# EUROPEAN PATENT APPLICATION

(11) **EP 4 631 457 A2**
(43) Date of publication of application: **15.10.2025**
(21) Application number: 25163943.1
(22) Date of filing: 14.03.2025
(51) Int. Cl.: A61B 34/20, G06T 7/00

(54) **INFORMATION PROCESSING APPARATUS AND INFORMATION PROCESSING PROGRAM**

(30) Priority: 18.03.2024 JP 2024042843
(71) Applicant: FUJI-FILM Corporation, Minato-ku Tokyo 106-8620 (JP)
(72) Inventor: CHIBA, Haruto, Tokyo (JP)
(74) Representative: Dehns Germany Partnerschaft mbB

(57) **Abstract**

A viewpoint of an endoscope in a living body is ascertained from an endoscopic image even in a case where an unknown structure region that makes it difficult to specify an object is included in the endoscopic image as compared to a case where the unknown structure region is not present.

An information processing apparatus (3) detects an unknown structure region from an endoscopic image (4), acquires a bronchial model generated in advance, estimates a viewpoint of an endoscope at a position where the endoscopic image has been captured, using the endoscopic image, the unknown structure region, and the bronchial model, and notifies of the estimated viewpoint of the endoscope.

## Description

### BACKGROUND OF THE INVENTION

### 1. Field of the Invention

The present disclosure relates to an information processing apparatus and an information processing program.

### 2. Description of the Related Art

For example, WO2019/130868A discloses an image processing apparatus that extracts feature points from each of an acquired endoscopic image and a three-dimensional image, acquires viewpoint information for obtaining a virtual endoscopic image having feature points matched with the feature points of the endoscopic image, generates a virtual endoscopic image based on the three-dimensional image and the viewpoint information, and displays the endoscopic image and the virtual endoscopic image on the same screen.

JP2011-173A discloses an endoscopy support system that stores volume data related to a luminal object having a lesion part and data of an endoscopic image related to the lesion part, generates a plurality of virtual endoscopic images based on the volume data, and displays a combination of images in which a similarity between the endoscopic image and the virtual endoscopic image has the maximum value on a display unit.

JP2012-50606A discloses an endoscopic image processing apparatus that acquires a plurality of endoscopic images and volume data, generates a plurality of virtual endoscopic images from the volume data, extracts an endoscopic image captured at a predetermined position and a comparative virtual endoscopic image virtually generated as being captured at a position corresponding to the predetermined position, associates the extracted images, and calculates a position of a pixel corresponding to each of a plurality of pixels constituting the endoscopic image captured at the predetermined position based on a three-dimensional position of each of a plurality of pixels constituting the comparative virtual endoscopic image.

### SUMMARY OF THE INVENTION

In endoscopy or the like, an information processing apparatus that estimates the position and posture of an endoscope in a living body from an endoscopic image and notifies a medical worker of a traveling direction of the endoscope such that the endoscope reaches a target location may be used.

In this case, in a case where the endoscopic image includes an unknown structure region which is a region that makes it difficult to specify an object as compared to a case where the unknown structure region is not present, the amount of information obtained from the endoscopic image may be reduced, and the accuracy of estimation of the position and posture of the endoscope may be reduced.

The present disclosure has been made in view of the above circumstances, and an object of the present disclosure is to provide an information processing apparatus and an information processing program that can ascertain a viewpoint of an endoscope in a living body from an endoscopic image even in a case where an unknown structure region is included in the endoscopic image.

According to a first aspect of the technology of the present disclosure, there is provided an information processing apparatus comprising: a detection unit that detects an unknown structure region from an endoscopic image of a living body; an acquisition unit that acquires a three-dimensional model of the living body generated in advance; an estimation unit that estimates, as a viewpoint of an endoscope, a posture of the endoscope at a position of the three-dimensional model corresponding to a position where the endoscopic image has been captured, using the endoscopic image, the unknown structure region, and the three-dimensional model; and a notification unit that notifies of the estimated viewpoint of the endoscope.

According to a second aspect of the technology of the present disclosure, the information processing apparatus according to the first aspect may further comprise a generation unit that generates a virtual endoscopic image of the living body in a case where the three-dimensional model is viewed from a predetermined position in the three-dimensional model. The estimation unit may input the endoscopic image, positional information of the unknown structure region, and the virtual endoscopic image generated from the three-dimensional model to an estimation model that has been subjected to machine learning in advance to output a viewpoint difference between the endoscopic image and the virtual endoscopic image for the endoscopic image, the positional information of the unknown structure region, and the virtual endoscopic image to acquire the viewpoint difference and may estimate the viewpoint of the endoscope from the viewpoint difference and a position at which the virtual endoscopic image has been obtained.

According to a third aspect of the technology of the present disclosure, in the information processing apparatus according to the second aspect, the estimation unit may estimate the viewpoint of the endoscope, using a pseudo virtual endoscopic image obtained by converting the endoscopic image into an expression format of the virtual endoscopic image generated from the three-dimensional model instead of the endoscopic image.

According to a fourth aspect of the technology of the present disclosure, in the information processing apparatus according to the third aspect, the estimation unit may estimate the viewpoint of the endoscope, using an image, on which the positional information of the unknown structure region has been superimposed, as the pseudo virtual endoscopic image.

According to a fifth aspect of the technology of the present disclosure, in the information processing apparatus according to the third aspect, the estimation unit may estimate the viewpoint of the endoscope, using the pseudo virtual endoscopic image, on which the positional information of the unknown structure region has been superimposed, as the endoscopic image and the positional information of the unknown structure region.

According to a sixth aspect of the technology of the present disclosure, in the information processing apparatus according to the second aspect, the estimation unit may estimate the viewpoint of the endoscope, using the endoscopic image on which the positional information of the unknown structure region has been superimposed.

According to a seventh aspect of the technology of the present disclosure, in the information processing apparatus according to the second aspect, the endoscopic image used for the machine learning of the estimation model may be an image generated from the virtual endoscopic image.

According to an eighth aspect of the technology of the present disclosure, in the information processing apparatus according to the second aspect, the estimation model may be a model that has been subjected to machine learning in advance to output the viewpoint difference for the endoscopic image, the positional information of the unknown structure region, the virtual endoscopic image, and supplementary information related to a position of the endoscope, and the estimation unit may input the endoscopic image, the positional information of the unknown structure region, the virtual endoscopic image, and the supplementary information to the estimation model to estimate the viewpoint of the endoscope.

According to a ninth aspect of the technology of the present disclosure, in the information processing apparatus according to the eighth aspect, the supplementary information may be a depth image corresponding to the endoscopic image.

According to a tenth aspect of the technology of the present disclosure, in the information processing apparatus according to the first aspect, the estimation unit may superimpose positional information of the unknown structure region on a depth image corresponding to the endoscopic image to generate a superimposed depth image, superimpose the positional information of the unknown structure region on virtual depth images at respective positions in the three-dimensional model to generate superimposed virtual depth images, and estimate the viewpoint of the endoscope from a position indicated by the superimposed virtual depth image most similar to the superimposed depth image among the superimposed virtual depth images at respective positions in the three-dimensional model.

According to an eleventh aspect of the technology of the present disclosure, in the information processing apparatus according to the first aspect, the estimation unit may generate the endoscopic image, on which positional information of the unknown structure region in the endoscopic image has been superimposed, and virtual endoscopic images of the living body in a case where the three-dimensional model is viewed from respective positions in the three-dimensional model, each of which has the positional information of the unknown structure region in the endoscopic image superimposed thereon, and may estimate the viewpoint of the endoscope from a position indicated by the virtual endoscopic image most similar to the endoscopic image, on which the positional information of the unknown structure region has been superimposed, among the virtual endoscopic images at respective positions in the three-dimensional model.

According to a twelfth aspect of the technology of the present disclosure, in the information processing apparatus according to any one of the first to eleventh aspects, the notification unit may notify of the unknown structure region detected from the endoscopic image.

According to a thirteenth aspect of the technology of the present disclosure, in the information processing apparatus according to the twelfth aspect, the notification unit may surround the unknown structure region in the endoscopic image with a frame to notify of the unknown structure region.

A fourteenth aspect of the technology of the present disclosure relates to the information processing apparatus according to the twelfth aspect, the notification unit may display an edge of the endoscopic image, whose distance from the unknown structure region is equal to or less than a predetermined value, in a form different from other edges of the endoscopic image.

According to a fifteenth aspect of the technology of the present disclosure, in the information processing apparatus according to the thirteenth or fourteenth aspect, the notification unit may notify of the unknown structure region in a case where a proportion of the unknown structure region to the endoscopic image is equal to or greater than a prescribed proportion.

According to a sixteenth aspect of the technology of the present disclosure, there is provided an information processing program causing a computer to execute a process comprising: detecting an unknown structure region from an endoscopic image of a living body; acquiring a three-dimensional model of the living body generated in advance; estimating, as a viewpoint of an endoscope, a posture of the endoscope at a position of the three-dimensional model corresponding to a position where the endoscopic image has been captured, using the endoscopic image, the unknown structure region, and the three-dimensional model; and notifying of the estimated viewpoint of the endoscope.

According to the present disclosure, even in a case where an unknown structure region is included in an endoscopic image, it is possible to ascertain a viewpoint of an endoscope in a living body from the endoscopic image.

### BRIEF DESCRIPTION OF THE DRAWINGS

Fig. 1 is a diagram showing an example of a configuration of an information processing system.
Fig. 2 is a diagram showing an example of an endoscopic image.
Fig. 3 is a diagram showing an example of an endoscopic image including an unknown structure region.
Fig. 4 is a diagram showing an example of a bronchial model.
Fig. 5 is a diagram showing an example of a virtual endoscopic image.
Fig. 6 is a diagram showing an example of a method for estimating a viewpoint of an endoscope.
Fig. 7 is a diagram showing an example of machine learning of an unknown structure region detection model.
Fig. 8 is a diagram showing an example of display of positional information of the unknown structure region.
Fig. 9 is a diagram showing another example of the display of the positional information of the unknown structure region.
Fig. 10 is a diagram showing an example of tracking of the viewpoint of the endoscope.
Fig. 11 is a diagram showing an example of machine learning of a viewpoint difference estimation model.
Fig. 12 is a diagram showing an example of an information processing apparatus configured by a computer.
Fig. 13 is a flowchart showing an example of a flow of an estimation process in a first embodiment.
Fig. 14 is a diagram showing an example of the display of the unknown structure region in the endoscopic image.
Fig. 15 is a diagram showing an example of a method for estimating a viewpoint of an endoscope in Modification Example 1.
Fig. 16 is a diagram showing an example of a method for estimating a viewpoint of an endoscope in Modification Example 2.
Fig. 17 is a diagram showing an example of a method for estimating a viewpoint of an endoscope in Modification Example 3.
Fig. 18 is a diagram showing an example of machine learning of a viewpoint difference estimation model in Modification Example 3.
Fig. 19 is a diagram showing another example of the machine learning of the viewpoint difference estimation model in Modification Example 3.
Fig. 20 is a diagram showing an example of a method for estimating a viewpoint of an endoscope in a second embodiment.
Fig. 21 is a flowchart showing an example of a flow of an estimation process in the second embodiment.
Fig. 22 is a diagram showing an example of a method for estimating a viewpoint of an endoscope to which supplementary information is added.

### DESCRIPTION OF THE PREFERRED EMBODIMENTS

Hereinafter, embodiments of the present disclosure will be described in detail with reference to the drawings. In addition, the same components and the same processes are denoted by the same reference numerals throughout the drawings, and a duplicate description thereof will be omitted. Dimensional ratios in the drawings are exaggerated for convenience of description and may be different from the actual ratios.

### First Embodiment

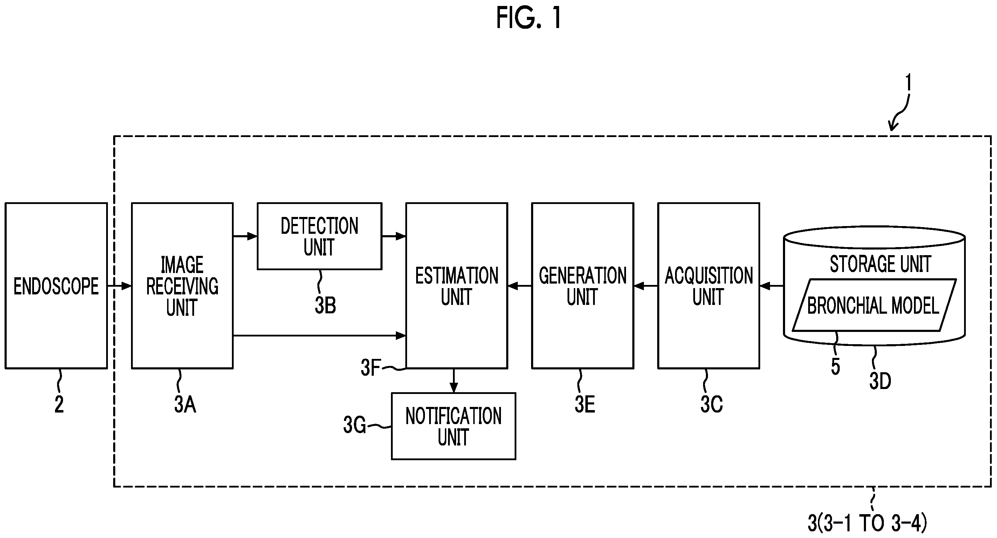

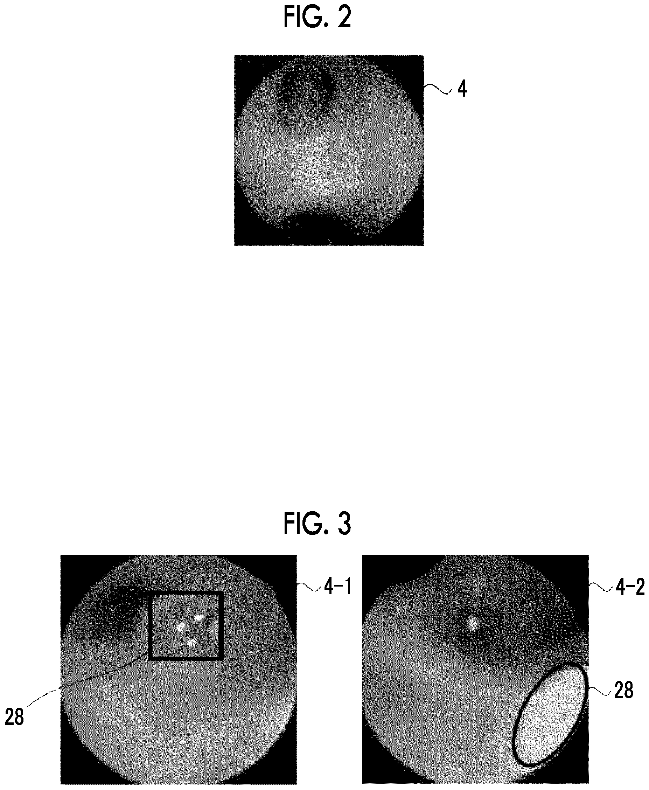

Fig. 1 is a diagram showing an example of a configuration of an information processing system 1 according to a first embodiment. As shown in Fig. 1, the information processing system 1 includes an endoscope 2 and an information processing apparatus 3. An imaging device, such as a camera, is provided at a distal end of the endoscope 2. The information processing system 1 transmits an image captured by the endoscope 2, that is, an endoscopic image 4 to the information processing apparatus 3. Fig. 2 is a diagram showing an example of the endoscopic image 4 captured by the endoscope 2.

There is no restriction on a location of a living body into which the endoscope 2 is inserted, and the endoscope 2 may be, for example, an endoscope 2 for a digestive organ or a ureteroscope. However, as an example, the endoscope 2 according to the embodiment of the present disclosure will be described as a bronchoscope that is inserted into a bronchus of a subject.

In a case where the endoscope 2 is inserted into the bronchus and advanced to a position to be examined, the bronchi have many bifurcations and have a similar shape. Therefore, in some cases, a navigation device is used that notifies a medical worker who operates the endoscope 2 of support information indicating the position and direction of the endoscope 2 in the bronchus and a direction in which the endoscope 2 is to be advanced to reach the position to be examined (hereinafter, referred to as a "target location").

The information processing apparatus 3 is an example of a navigation device that performs navigation of the endoscope 2. The information processing apparatus 3 includes each functional unit of an image receiving unit 3A, a detection unit 3B, an acquisition unit 3C, a storage unit 3D, a generation unit 3E, an estimation unit 3F, and a notification unit 3G.

The image receiving unit 3A receives the endoscopic image 4 from the endoscope 2.

The detection unit 3B detects an unknown structure region from the endoscopic image 4 received by the image receiving unit 3A. The unknown structure region means a region in the endoscopic image 4 that is not desired to be used for estimating the position and posture of the endoscope 2 in the execution of an estimation process of estimating the position and posture of the endoscope 2 using the endoscopic image 4 by the information processing apparatus 3.

Examples of the unknown structure region include a region having bubbles attached thereto, a region in which the endoscope 2 is out of focus, a region in which the image is blocked by a substance attached to a lens of the endoscope 2, a reflection region in which light illumination is reflected, a whiteout region, and a blackout region. That is, the unknown structure region is a general term for a region that makes it difficult to specify an object.

Fig. 3 is a diagram showing an example of the endoscopic image 4 including the unknown structure region. In a region indicated by a frame 28 of an endoscopic image 4-1 shown in Fig. 3, bubbles attached to the bronchus which is an example of the unknown structure region are recognized. In addition, in a region indicated by a frame 28 of an endoscopic image 4-2, the reflection of illumination light is recognized.

In the estimation of the position and posture of the endoscope 2 using the endoscopic image 4 by the information processing apparatus 3, in a case where the estimation is performed using information of the unknown structure region that does not correctly represent the shape or surface pattern of the bronchus, there is a concern that the accuracy of estimation will be reduced, as compared to a case where the estimation is performed using the endoscopic image 4 in which the unknown structure region is not present.

Therefore, the detection unit 3B detects the unknown structure region from the endoscopic image 4 and specifies the position of the unknown structure region in the endoscopic image 4 to reduce the influence of the unknown structure region on the estimation of the position and posture of the endoscope 2 in the subsequent estimation process performed by the information processing apparatus 3.

**In** addition, the posture of the endoscope 2 is a direction in which the endoscope 2 is facing. Since an imaging range of the endoscopic image 4 captured by the endoscope 2 is determined by the position and posture of the endoscope 2, hereinafter, the position and posture of the endoscope 2 is referred to as a "viewpoint of the endoscope 2".

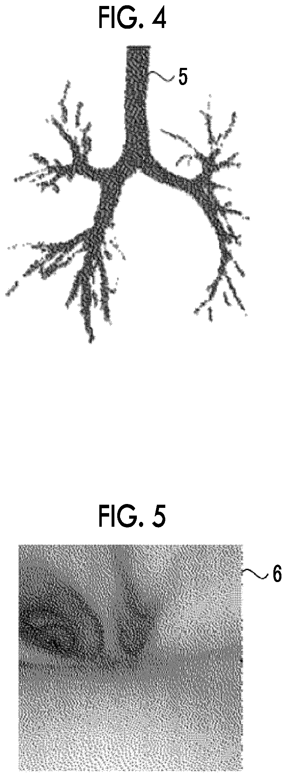

Meanwhile, the bronchial model 5 representing the shape of the bronchus of the subject is used to notify the medical worker of a traveling direction of the endoscope 2. The bronchial model 5 is an example of a three-dimensional model that is generated in advance by, for example, computed tomography (CT) before endoscopy. Fig. 4 shows an example of the bronchial model 5. The bronchial model 5 is generated in advance for each subject and is stored in advance in the storage unit 3D.

The information processing apparatus 3 shown in Fig. 1 includes the storage unit 3D. However, the information processing apparatus 3 does not necessarily include the storage unit 3D. For example, an external apparatus other than the information processing apparatus 3, such as a data server, may be used as the storage unit 3D.

The acquisition unit 3C shown in Fig. 1 acquires the bronchial model 5 of the subject to be examined from the storage unit 3D.

Further, the generation unit 3E generates an image in a case where the bronchial model 5 is viewed from a predetermined position in the bronchial model 5, that is, the virtual endoscopic image 6, using the bronchial model 5 acquired by the acquisition unit 3C. For example, a position where the distal end of the endoscope 2 is estimated to be present is used as the predetermined position in the bronchial model 5. Fig. 5 is a diagram showing an example of the virtual endoscopic image 6 generated by the generation unit 3E using the bronchial model 5.

Since the bronchial model 5 is a three-dimensional model generated from data representing the shape of the bronchus of the subject, bubbles or foreign substances are not attached to the bronchial model 5 unlike the actual bronchus of the subject. In addition, since the virtual endoscopic image 6 is generated by data processing using the bronchial model 5, the virtual endoscopic image 6 is not out of focus, illumination light is not reflected, and whiteout or blackout does not occur. That is, the virtual endoscopic image 6 is an image that faithfully reproduces how the shape of the bronchial model 5 looks in a case where the bronchial model 5 is viewed from the designated viewpoint. Since the viewpoint of the endoscope 2 can be freely set in the bronchial model 5, the generation unit 3E can generate the virtual endoscopic image 6 from any viewpoint.

The estimation unit 3F shown in Fig. 1 estimates, as the viewpoint of the endoscope 2, the posture of the endoscope at the position of the bronchial model 5 corresponding to the position where the endoscopic image 4 has been captured, using the endoscopic image 4, the unknown structure region, and the virtual endoscopic image 6 generated from the bronchial model 5. In addition, the estimation unit 3F determines the traveling direction of the endoscope 2 for reaching the target location based on the estimated viewpoint of the endoscope 2. Further, the estimation unit 3F causes the generation unit 3E to generate the virtual endoscopic image 6 obtained from the estimated viewpoint of the endoscope 2. Furthermore, a method for estimating the viewpoint of the endoscope 2 in the estimation unit 3F will be described in detail below.

The notification unit 3G notifies the medical worker of the viewpoint of the endoscope 2 estimated by the estimation unit 3F. Specifically, the notification unit 3G notifies of the estimated position and posture of the endoscope 2 in the bronchus. **In** addition, the notification unit 3G notifies the medical worker of the virtual endoscopic image 6 obtained from the viewpoint of the endoscope 2 estimated by the estimation unit 3F together with the endoscopic image 4. Further, the notification unit 3G notifies the medical worker of the traveling direction of the endoscope 2 for reaching the target location.

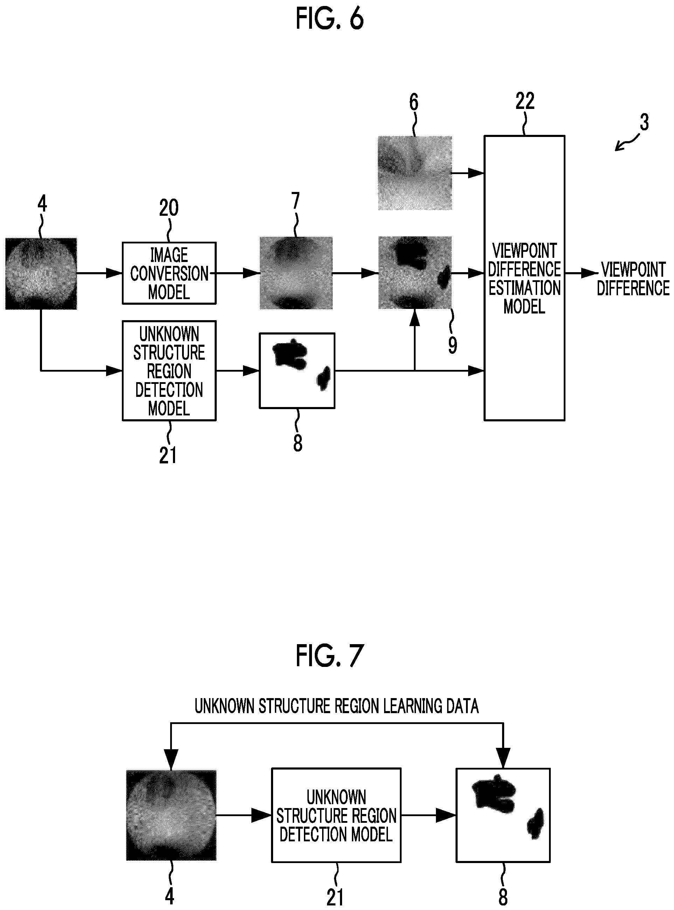

Next, a method for estimating the viewpoint of the endoscope 2 in the estimation unit 3F will be described. Fig. 6 is a diagram showing an example of the method for estimating the viewpoint of the endoscope 2. The estimation unit 3F estimates the viewpoint of the endoscope 2 using a viewpoint difference estimation model 22 that has been subjected to machine learning in advance to output the viewpoint difference between the endoscopic image 4 and the virtual endoscopic image 6 for the endoscopic image 4, the positional information of the unknown structure region in the endoscopic image 4, and the virtual endoscopic image 6 in a case where the bronchial model 5 is viewed from the designated viewpoint. **In** addition, the endoscopic image 4 input to the viewpoint difference estimation model 22 includes an image obtained from the endoscopic image 4 in addition to the endoscopic image 4.

As can be seen from the fact that the viewpoint difference estimation model 22 outputs the viewpoint difference between the input endoscopic image 4 and the input virtual endoscopic image 6, the virtual endoscopic image 6 input to the viewpoint difference estimation model 22 may be the virtual endoscopic image 6 obtained from a viewpoint different from the viewpoint at which the endoscopic image 4 has been captured.

As preparation for estimating the viewpoint of the endoscope 2, the detection unit 3B inputs the endoscopic image 4 to an unknown structure region detection model 21 to detect the unknown structure region from the endoscopic image 4 and acquires the positional information of the unknown structure region in the endoscopic image 4. The positional information of the unknown structure region in the endoscopic image 4 is represented as, for example, a mask image 8 in which a position corresponding to the location where the unknown structure region is present is painted. That is, the painted location in the mask image 8 indicates a region in the endoscopic image 4 that is not desired to be used for estimating the viewpoint of the endoscope 2.

The unknown structure region detection model 21 is, for example, a model generated in advance by machine learning and is stored in the storage unit 3D in advance.

Fig. 7 is a diagram showing an example of the machine learning of the unknown structure region detection model 21. A plurality of learning data items (hereinafter, referred to as "unknown structure region learning data items"), in which a mask image 8 in which a position corresponding to the location where the unknown structure region of the endoscopic image 4 is present is painted is associated with the endoscopic image 4 captured in advance, are prepared in advance. In addition, the machine learning of the unknown structure region detection model 21 is performed such that, in a case where the endoscopic image 4 included in the unknown structure region learning data is input, the unknown structure region detection model 21 outputs the mask image 8 associated with the input endoscopic image 4. That is, in the unknown structure region learning data, the endoscopic image 4 is input data, and the mask image 8 is training data. For example, a creator of the unknown structure region learning data views the endoscopic image 4 and designates a location that is considered to be the unknown structure region to generate the mask image 8 in the unknown structure region learning data.

For example, a convolutional neural network (CNN) is used as the unknown structure region detection model 21. For example, deep learning is used as the machine learning method.

**In** the present disclosure, an example in which the unknown structure region is detected from the endoscopic image 4 using the unknown structure region detection model 21 will be described. However, the information processing apparatus 3 may detect the unknown structure region from the endoscopic image 4 using other methods without using the estimation model such as the unknown structure region detection model 21 generated by machine learning. For example, in the endoscopic image 4, a region in which a predetermined number or more of pixels having pixel values included in a range of pixel values corresponding to a predetermined color, such as white and black, are continuous may be detected as the unknown structure region. **In** addition, frequency analysis may be performed on the endoscopic image 4, and a region including, for example, a prescribed number of high-frequency components or low-frequency components equal to or greater than a threshold value may be detected as the unknown structure region.

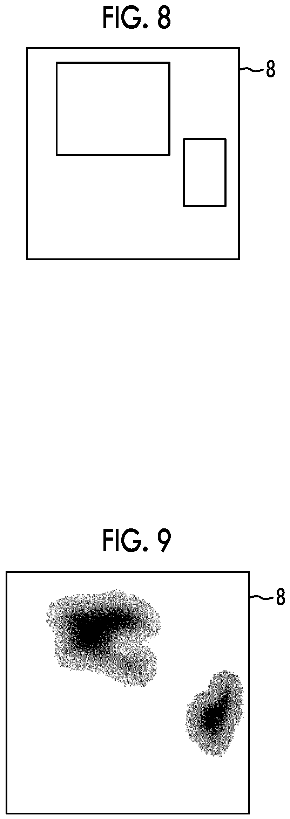

Further, the positional information of the unknown structure region in the endoscopic image 4 is not necessarily displayed by painting like the mask image 8. Figs. 8 and 9 are diagrams showing examples of the display of the positional information of the unknown structure region. Fig. 8 is a diagram showing an example of the display using a bounding box that surrounds the position of the unknown structure region with a rectangle. Fig. 9 is a diagram showing an example of the display using a heat map in which the position of the unknown structure region is represented by a difference in color or density. As described above, the positional information of the unknown structure region is also indicated in a format other than the mask image 8.

In Fig. 6, the estimation unit 3F inputs the endoscopic image 4 to an image conversion model 20 to convert the endoscopic image 4 into a pseudo virtual endoscopic image 7.

The pseudo virtual endoscopic image 7 is an image obtained by converting the endoscopic image 4 into an expression format that looks like the virtual endoscopic image 6. Specifically, the pseudo virtual endoscopic image 7 is, for example, an image obtained by removing a pattern of a bronchial wall (referred to as a "texture of a bronchus") due to the state of blood vessels or tissues and the like or the unknown structure region from the endoscopic image 4 such that the shape of the bronchus is more easily ascertained from the image than the endoscopic image 4.

The image conversion model 20 is a model generated in advance using, for example, a generative adversarial network (GAN) trained to output the pseudo virtual endoscopic image 7 similar to the virtual endoscopic image 6 from the endoscopic image 4 and is stored in the storage unit 3D in advance.

Furthermore, the estimation unit 3F superimposes the mask image 8 generated by the unknown structure region detection model 21 on the pseudo virtual endoscopic image 7 generated by the image conversion model 20 to generate a mask superimposed pseudo virtual image 9. The mask superimposed pseudo virtual image 9 is an example of the image obtained from an endoscopic image 4.

The estimation unit 3F inputs the virtual endoscopic image 6, the mask image 8, and the mask superimposed pseudo virtual image 9 obtained as described above to the viewpoint difference estimation model 22 to acquire the viewpoint difference between the virtual endoscopic image 6 and the endoscopic image 4 which is the generation source of the mask superimposed pseudo virtual image 9. Since the virtual endoscopic image 6 is an image generated from the bronchial model 5, the viewpoint of the virtual endoscopic image 6 is known to the estimation unit 3F. Furthermore, since the viewpoint difference between the endoscopic image 4 and the virtual endoscopic image 6 is obtained from the viewpoint difference estimation model 22, the estimation unit 3F can estimate the viewpoint of the endoscope 2 from the viewpoint of the virtual endoscopic image 6 and the viewpoint difference between the endoscopic image 4 and the virtual endoscopic image 6.

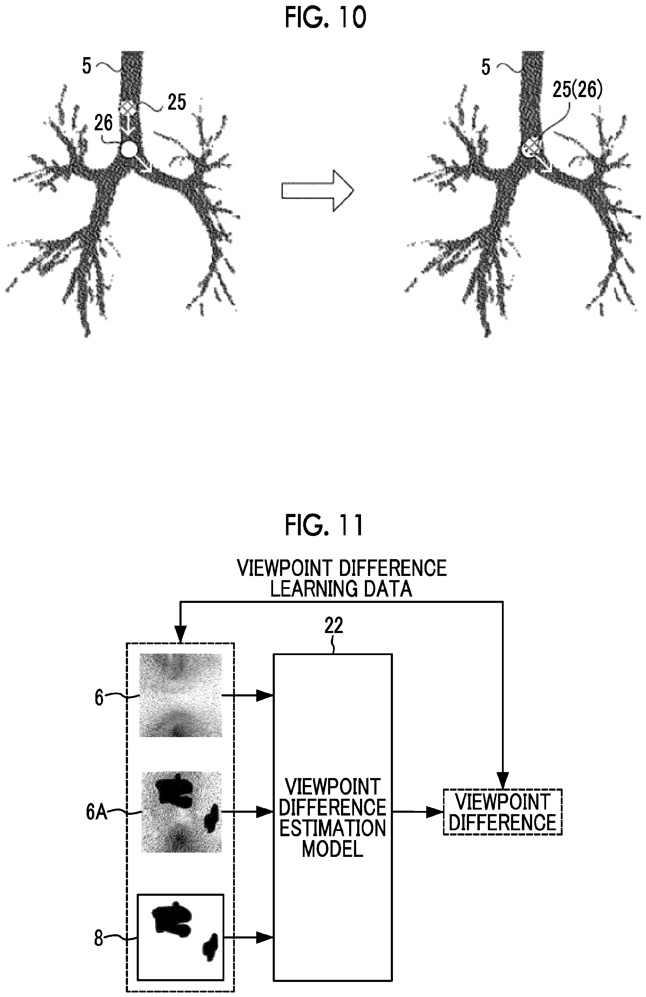

Fig. 10 is a diagram showing an example of the tracking of the viewpoint of the endoscope 2. **In** Fig. 10, a mark 25 indicates a predetermined position in the bronchial model 5 (for example, a schematic position where the endoscope 2 is considered to be present), and a mark 26 indicates an actual position of the endoscope 2. The estimation unit 3F can move the position of the mark 25 to the position of the mark 26 from the estimated viewpoint difference and take the same posture as the endoscope 2 whose position is indicated by the mark 26 on the bronchial model 5.

**In** a case where the viewpoint of the endoscope 2 indicated by the endoscopic image 4 is known, the virtual endoscopic image 6 obtained from the same viewpoint as the endoscope 2 is generated from the bronchial model 5, and it is possible to notify the medical worker of the generated virtual endoscopic image 6 together with the endoscopic image 4. That is, the information processing apparatus 3 can display the virtual endoscopic image 6 corresponding to the viewpoint of the endoscope 2 with the movement of the endoscope 2.

In addition, in a case where the viewpoint of the endoscope 2 indicated by the endoscopic image 4 is known, the information processing apparatus 3 can specify the traveling direction of the endoscope 2 for reaching the target location using the bronchial model 5.

**In** addition, the reason for using the image close to the virtual endoscopic image 6, such as the mask superimposed pseudo virtual image 9, as the input of the viewpoint difference estimation model 22 instead of the endoscopic image 4 is to consider ease of the preparation of learning data (hereinafter, referred to as "viewpoint difference learning data") used for machine learning of the viewpoint difference estimation model 22.

For example, two endoscopic images 4 whose viewpoints deviate from each other and information related to the viewpoint difference between the endoscopic images 4 are used to perform the machine learning of the viewpoint difference estimation model 22. However, the endoscope 2 is being miniaturized such that the endoscope 2 can be inserted into as many bronchi as possible due to the characteristics of the endoscope 2. For example, it is difficult to separately attach sensors for measuring the viewpoint of the endoscope 2, such as a gyro sensor and a motion sensor. **In** addition, since the endoscope 2 is inserted into the body, it is possible to ascertain the viewpoint of the endoscope 2 only through the endoscopic image 4 with a limited angle of view, and it is also difficult to acquire the viewpoint difference from the endoscopic image 4.

In contrast, as described above, the viewpoint of the endoscope 2 can be freely set in the bronchial model 5. Since the bronchial model 5 is a three-dimensional model based on examination data of CT or the like, a correct viewpoint difference can be acquired by numerical calculation.

Therefore, in a case where a combination of two virtual endoscopic images 6 whose viewpoints deviate from each other and information related to the viewpoint difference between the virtual endoscopic images 6 is used as the viewpoint difference learning data, it is possible to more easily prepare a larger amount of viewpoint difference training data, as compared to a case where a combination of two endoscopic images 4 whose viewpoints deviate from each other and information related to the viewpoint difference between the endoscopic images 4 is used as the viewpoint difference learning data.

For the above reason, two virtual endoscopic images 6 having different viewpoints are used as the input data in the viewpoint difference learning data of the viewpoint difference estimation model 22. Specifically, the positional information of the unknown structure region that is randomly set is superimposed on one virtual endoscopic image 6 as the input data such that the viewpoint difference between the two virtual endoscopic images 6 can be estimated even in a case where the information of a region corresponding to the unknown structure region is missing. Further, the mask image 8 indicating the positional information of the unknown structure region is included in the input data in the viewpoint difference learning data.

The viewpoint difference estimation model 22 is a model generated in advance by the machine learning and is stored in the storage unit 3D in advance.

Fig. 11 is a diagram showing an example of the machine learning of the viewpoint difference estimation model 22.

As described above, a plurality of viewpoint difference learning data items in which the viewpoint difference between two virtual endoscopic images 6 is associated with the two virtual endoscopic images 6, one of which the positional information of the unknown structure region has been superimposed on, and the mask image 8 indicating the positional information of the unknown structure region are prepared in advance. In Fig. 11, a virtual endoscopic image 6A indicates the virtual endoscopic image 6 on which the positional information of the unknown structure region is superimposed.

In addition, the machine learning of the viewpoint difference estimation model 22 is performed such that, in a case where the virtual endoscopic images 6 and 6A and the mask image 8 included in the viewpoint difference learning data are input, the viewpoint difference estimation model 22 outputs the viewpoint difference associated with each input. For example, a CNN or the like is used as the viewpoint difference estimation model 22. For example, deep learning is used as the machine learning method.

That is, in the viewpoint difference learning data, the virtual endoscopic images 6 and 6A and the mask image 8 are input data, and the viewpoint difference is training data.

In addition, the positional information of the unknown structure region in the virtual endoscopic image 6 is not necessarily displayed by painting like the mask image 8, similarly to the positional information of the unknown structure region in the endoscopic image 4. In a case where the position of the unknown structure region is known, the positional information of the unknown structure region may be indicated in any format.

In addition, the machine learning of the viewpoint difference estimation model 22 may be performed using the range of the bronchial model 5 (referred to as a "partial bronchial model 5A") that is visible from each viewpoint instead of the virtual endoscopic image 6. That is, the virtual endoscopic image 6 may be expressed as three-dimensional data. In this case, in the estimation of the viewpoint of the endoscope 2 shown in Fig. 6, the partial bronchial model 5A at the designated viewpoint is used instead of the virtual endoscopic image 6.

The machine learning of various models, such as the image conversion model 20, the unknown structure region detection model 21, and the viewpoint difference estimation model 22, and the generation of the bronchial model 5 are not necessarily performed by the information processing apparatus 3 and may be performed by another computer having a higher processing capacity than the information processing apparatus 3. In this case, the information processing apparatus 3 acquires various models generated by another computer from another computer and uses the various models.

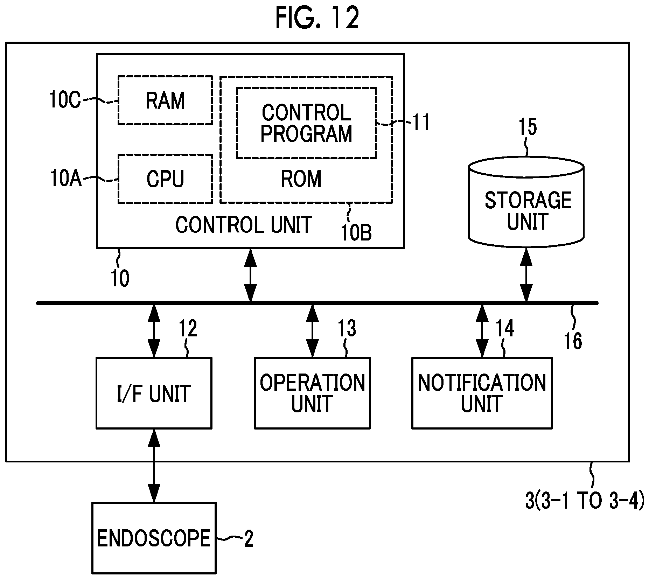

The information processing apparatus 3 shown in Fig. 1 is configured by, for example, a computer. Fig. 12 is a diagram showing an example of a configuration of the information processing apparatus 3 configured by the computer.

As shown in Fig. 12, the information processing apparatus 3 comprises a control unit 10, an interface (I/F) unit 12, an operation unit 13, a notification unit 14, and a storage unit 15. The control unit 10, the I/F unit 12, the operation unit 13, the notification unit 14, and the storage unit 15 are connected to each other via a bus 16 such that they can transmit and receive various types of information.

The control unit 10 controls the operation of the information processing apparatus 3 based on an instruction from the medical worker. The control unit 10 is an example of a processor and comprises a central processing unit (CPU) 10A that is in charge of processes of each functional unit of the information processing apparatus 3 shown in Fig. 1, a read only memory (ROM) 10B, and a random access memory (RAM) 10C. The ROM 10B stores in advance various programs including a control program 11 that is read by the CPU 10A to estimate the viewpoint of the endoscope 2 and various parameters that are referred to by the CPU 10A to control the operation of the information processing apparatus 3. The RAM 10C is used as a temporary work area of the CPU 10A.

The I/F unit 12 transmits and receives various types of information to and from the endoscope 2 using wireless communication or wired communication. The information processing apparatus 3 acquires the endoscopic image 4 from the endoscope 2 via the I/F unit 12.

The operation unit 13 is used, for example, by the medical worker to input instructions, various types of information, and the like related to the estimation of the viewpoint of the endoscope 2. There is no restriction on the operation form of the operation unit 13. For example, it is possible to receive the operations by a switch, a touch panel, a touch pen, a keyboard, a mouse, and the like.

The notification unit 14 notifies the medical worker of the information processed by the control unit 10, such as the endoscopic image 4, the virtual endoscopic image 6, and the information related to the estimation of the viewpoint of the endoscope 2.

The notification of the information is to enable the medical worker to recognize the information. Therefore, for example, all of a form in which information is displayed on a display (not shown) which is an example of the display device, a form in which information is printed on a recording medium, such as paper, by an image forming apparatus (not shown), and a form in which information is notified by voice through a speaker (not shown) are examples of the notification of the information by the notification unit 14. In addition, a form in which information is transmitted through the I/F unit 12 is also an example of the notification of the information. The information processing apparatus 3 according to the embodiment of the present disclosure notifies of the information by the display of the information on the display and by voice notification.

The storage unit 15 stores, for example, the bronchial model 5, and various models such as the image conversion model 20, the unknown structure region detection model 21, and the viewpoint difference estimation model 22. The storage unit 15 is an example of a storage device that retains stored information even in a case where power supplied to the storage unit 15 is cut off. For example, a semiconductor memory, such as a solid state drive (SSD), is used as the storage unit 15. However, a hard disk may be used.

Next, a process of the information processing apparatus 3 that estimates the viewpoint of the endoscope 2 will be described in detail.

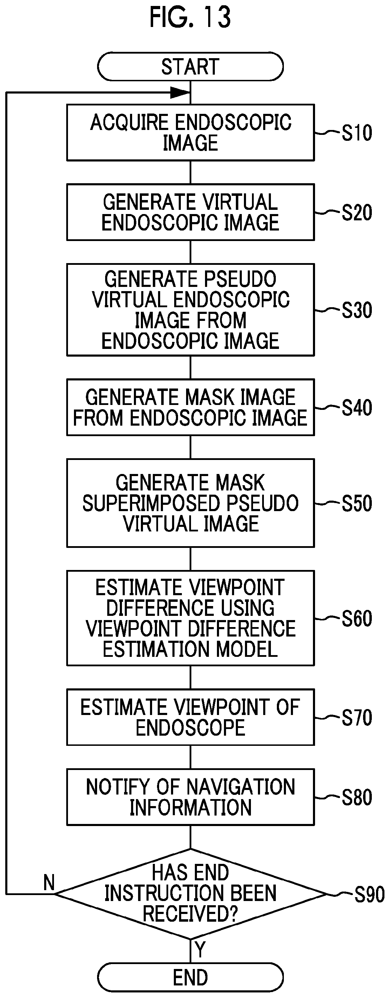

Fig. 13 is a flowchart showing an example of a flow of an estimation process executed by the information processing apparatus 3 in a case where an instruction to start the estimation of the viewpoint of the endoscope 2 is received by the operation of the operation unit 13 by the medical worker. The CPU 10A of the information processing apparatus 3 reads the control program 11 from the ROM 10B and executes the estimation process. The control program 11 is an example of an information processing program according to the embodiment of the present disclosure.

Further, it is assumed that the medical worker sets the initial viewpoint of the endoscope 2 in the bronchial model 5 in advance. The initial viewpoint of the endoscope 2 set by the medical worker may be a viewpoint different from the actual viewpoint of the endoscope 2. Alternatively, the CPU 10A may randomly set the initial viewpoint in the bronchial model 5.

In addition, it is assumed that the storage unit 15 stores the bronchial model 5 of the subject, the image conversion model 20, the unknown structure region detection model 21, and the viewpoint difference estimation model 22 in advance.

First, in Step S10, the CPU 10A acquires the endoscopic image 4 from the endoscope 2 via the I/F unit 12. The endoscopic image 4 may be a color image or a monochrome image.

In Step S20, the CPU 10A generates the virtual endoscopic image 6 in which the bronchial model 5 is viewed from the viewpoint set for the bronchial model 5.

In Step S30, the CPU 10A inputs the endoscopic image 4 acquired by the process in Step S10 to the image conversion model 20 to generate the pseudo virtual endoscopic image 7. In a case where the input data used for the machine learning of the viewpoint difference estimation model 22 is the virtual endoscopic image 6 and the endoscopic image 4 is input to the viewpoint difference estimation model 22 without any change, the accuracy of estimation of the viewpoint difference is reduced as compared to a case where the virtual endoscopic image 6 is input. Therefore, the CPU 10A converts the endoscopic image 4 into the pseudo virtual endoscopic image 7.

In Step S40, the CPU 10A inputs the endoscopic image 4 acquired by the process in Step S10 to the unknown structure region detection model 21 to generate the mask image 8.

In Step S50, the CPU 10A superimposes the mask image 8 generated by the process in Step S40 on the virtual endoscopic image 6 generated by the process in Step S20 to generate the mask superimposed pseudo virtual image 9.

In Step S60, the CPU 10A inputs the virtual endoscopic image 6 generated by the process in Step S20, the mask image 8 generated by the process in Step S40, and the mask superimposed pseudo virtual image 9 generated by the process in Step S50 to the viewpoint difference estimation model 22 to estimate the viewpoint difference between the endoscopic image 4 acquired by the process in Step S10 and the virtual endoscopic image 6 generated by the process in Step S20.

In Step S70, the CPU 10A reflects the viewpoint difference estimated by the process in Step S60 in the viewpoint set in the bronchial model 5 to estimate the viewpoint of the endoscope 2.

In Step S80, the CPU 10A sets the viewpoint of the endoscope 2 estimated by the process in Step S70 in the bronchial model 5 to update the viewpoint of the endoscope 2 in the bronchial model 5. In addition, the CPU 10A generates the virtual endoscopic image 6 in a case where the bronchial model 5 is viewed from the updated viewpoint of the endoscope 2 in the bronchial model 5 and displays the generated virtual endoscopic image 6 side by side with, for example, the endoscopic image 4 acquired by the process in Step S10 on the display. Therefore, the virtual endoscopic image 6 corresponding to the viewpoint of the endoscope 2 is displayed according to a change in the viewpoint of the endoscope 2.

In a case where the unknown structure region is detected from the endoscopic image 4, the CPU 10A may display the unknown structure region in the endoscopic image 4 such that the position of the unknown structure region is known.

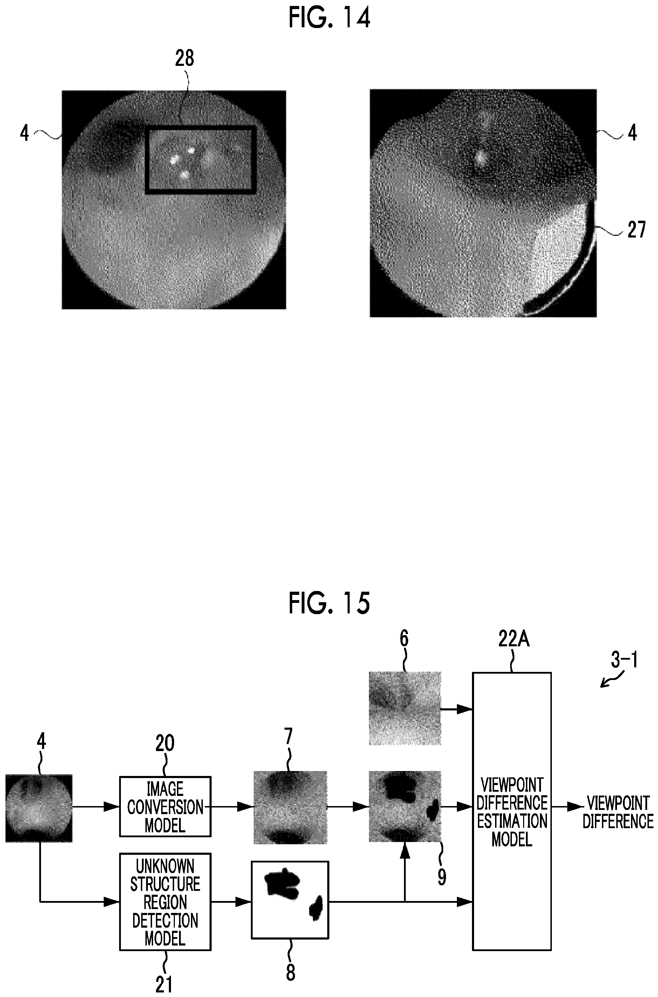

Fig. 14 is a diagram showing an example of the display of the unknown structure region in the endoscopic image 4. In a case where the unknown structure region is painted as in the mask image 8, it is not possible to visually discriminate the cause of the detection of the unknown structure region. Therefore, as shown in Fig. 14, the CPU 10A surrounds the unknown structure region with a frame 28. In addition, the CPU 10A may display a highlight line 27 along the edge of the endoscopic image 4 such that an edge of the endoscopic image 4 whose distance from the unknown structure region is equal to or less than a predetermined value is displayed in a form different from another edge of the endoscopic image 4.

The medical worker who has been notified of the presence of the unknown structure region may examine the cause of the detection of the unknown structure region to suppress the occurrence of the unknown structure region. In a case where the unknown structure region is caused by the adhesion of bubbles or foreign substances, it is possible to remove the unknown structure region using, for example, forceps or water supply. In addition, in a case where the unknown structure region is caused by the reflection of illumination light, for example, the unknown structure region can be removed by reducing the amount of illumination light.

The information processing apparatus 3 can estimate the viewpoint of the endoscope 2 even in a case where the endoscopic image 4 includes the unknown structure region. However, as the number of unknown structure regions is smaller, the accuracy of estimation is more improved. Therefore, the information processing apparatus 3 notifies the medical worker of the position of the detected unknown structure region to support the operation of the medical worker removing the cause of the occurrence of the unknown structure region.

In addition, as the area of the unknown structure region is smaller, the influence on the estimation of the viewpoint of the endoscope 2 is smaller. In a case where the position of the unknown structure region is notified regardless of the area of the detected unknown structure region, even though a treatment to remove the cause of the occurrence of the unknown structure region is performed, this does not contribute to improving the accuracy of estimation of the viewpoint of the endoscope 2 and may impose an extra burden on the medical worker. Therefore, in a case where the proportion of the unknown structure region to the endoscopic image 4 is equal to or greater than a prescribed proportion, the CPU 10A may be configured to notify of the position of the unknown structure region in the endoscopic image 4.

In addition, the CPU 10A searches for a route from the updated viewpoint of the endoscope 2 to the target location using the bronchial model 5. For example, a known search method, such as a binary search method, can be used to search for the route to the target location.

The CPU 10A notifies the medical worker of the traveling direction of the endoscope 2, such as a "right direction" or a "downward direction", by voice through the speaker based on the search result of the route.

In Step S90 shown in Fig. 13, the CPU 10A determines whether or not an instruction to end the estimation process has been received from the user. In a case where the end instruction has not been received, the CPU 10A proceeds to Step S10 and acquires a new endoscopic image 4 from the endoscope 2. That is, the information processing apparatus 3 estimates the viewpoint of the endoscope 2 with the movement of the endoscope 2 and notifies the medical worker of the navigation information of the endoscope 2 until the end instruction is received from the user.

On the other hand, in a case where the end instruction has been received from the user, the CPU 10A ends the estimation process shown in Fig. 13.

As described above, according to the information processing apparatus 3 of the first embodiment, the viewpoint of the endoscope 2 is estimated by the viewpoint difference estimation model 22 that has been subjected to machine learning using the viewpoint difference learning data in which the presence of the unknown structure region has been reflected in advance. Therefore, even in a case where the endoscopic image 4 includes the unknown structure region, it is possible to estimate the viewpoint of the endoscope 2 using the information of a region other than the unknown structure region. Therefore, even in a case where the endoscopic image 4 includes the unknown structure region, the information processing apparatus 3 can notify the medical worker of the traveling direction of the endoscope 2 such that the endoscope 2 reaches the target location.

### Modification Example 1 of Information Processing Apparatus 3

The example in which the virtual endoscopic image 6, the mask image 8, and the mask superimposed pseudo virtual image 9 are input to the viewpoint difference estimation model 22 to estimate the viewpoint difference between the endoscopic image 4 and the virtual endoscopic image 6 has been described above. However, the input data to the viewpoint difference estimation model 22 used for estimating the viewpoint difference is not limited to the above example.

For example, the mask superimposed pseudo virtual image 9 also includes the positional information of the unknown structure region in the endoscopic image 4 and the information of the endoscopic image 4. Therefore, the information processing apparatus 3 can use the mask superimposed pseudo virtual image 9 instead of the endoscopic image 4 and the mask image 8.

Fig. 15 is a diagram showing an example of a method for estimating the viewpoint of the endoscope 2 in an information processing apparatus 3-1 which is a modification example of the information processing apparatus 3. An example of a functional configuration of the information processing apparatus 3-1 is the same as the example of the functional configuration of the information processing apparatus 3 shown in Fig. 1, and the information processing apparatus 3-1 is configured by the computer shown in Fig. 12.

In a case where the virtual endoscopic image 6 and the mask superimposed pseudo virtual image 9 are input to a viewpoint difference estimation model 22A shown in Fig. 15, the viewpoint difference estimation model 22A outputs the viewpoint difference between the virtual endoscopic image 6 and the endoscopic image 4 which is a generation source of the mask superimposed pseudo virtual image 9.

For this, a plurality of viewpoint difference learning data items, in which the viewpoint difference between the virtual endoscopic image 6A having the positional information of the unknown structure region superimposed thereon and the virtual endoscopic image 6 having a viewpoint different from that of the virtual endoscopic image 6A is associated with the virtual endoscopic image 6 and the virtual endoscopic image 6A, may be prepared in advance, and the machine learning of the viewpoint difference estimation model 22A may be performed such that, in a case where the virtual endoscopic images 6 and 6A included in the viewpoint difference learning data are input, the viewpoint difference estimation model 22A outputs the viewpoint difference associated with each input. That is, in the viewpoint difference learning data of the viewpoint difference estimation model 22A, the virtual endoscopic images 6 and 6A are input data, and the viewpoint difference is training data.

In this case, since the number of input dimensions of the viewpoint difference estimation model 22A is smaller than the number of input dimensions of the viewpoint difference estimation model 22, the time required for the machine learning of the viewpoint difference estimation model 22A may be shorter than the time required for the machine learning of the viewpoint difference estimation model 22.

### Modification Example 2 of Information Processing Apparatus 3

Each of the mask image 8 and the mask superimposed pseudo virtual image 9 includes the positional information of the unknown structure region in the endoscopic image 4. Therefore, in the above-described Modification Example 1 of the information processing apparatus 3, the example has been described in which the viewpoint difference between the endoscopic image 4 and the virtual endoscopic image 6 is estimated using the mask superimposed pseudo virtual image 9 including the positional information of the unknown structure region, without using the mask image 8. However, even in a case where an image obtained by removing the positional information of the unknown structure region from the mask superimposed pseudo virtual image 9, that is, the pseudo virtual endoscopic image 7 is used instead of leaving the mask image 8, it is possible to estimate the viewpoint difference because the positional information of the unknown structure region is included in the mask image 8.

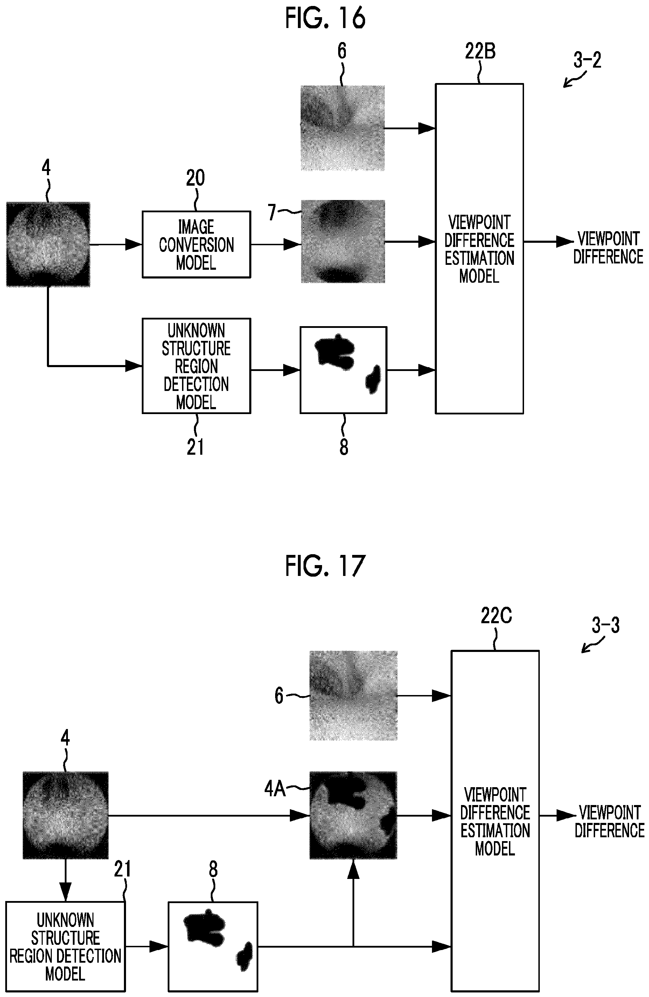

Fig. 16 is a diagram showing an example of a method for estimating the viewpoint of the endoscope 2 in an information processing apparatus 3-2 which is a modification example of the information processing apparatus 3. An example of a functional configuration of the information processing apparatus 3-2 is the same as the example of the functional configuration of the information processing apparatus 3 shown in Fig. 1, and the information processing apparatus 3-2 is configured by the computer shown in Fig. 12.

In a case where the virtual endoscopic image 6, the pseudo virtual endoscopic image 7, and the mask image 8 are input, a viewpoint difference estimation model 22B shown in Fig. 16 outputs the viewpoint difference between the virtual endoscopic image 6 and the endoscopic image 4 which is the generation source of the pseudo virtual endoscopic image 7.

For this, a plurality of viewpoint difference learning data items, in which the viewpoint difference of each of two virtual endoscopic images 6 having different viewpoints is associated with the two virtual endoscopic images 6 and the mask image 8 in which the positional information of the unknown structure region has been randomly set, may be prepared in advance, and the machine learning of the viewpoint difference estimation model 22B may be performed such that, in a case where each of the virtual endoscopic images 6 and the mask image 8 included in the viewpoint difference learning data are input, the viewpoint difference estimation model 22B outputs the viewpoint difference associated with each input. That is, in the viewpoint difference learning data of the viewpoint difference estimation model 22B, the two virtual endoscopic images 6 having different viewpoints and the mask images 8 are input data, and the viewpoint difference is training data.

In this case, since the superimposition of the mask image 8 on the pseudo virtual endoscopic image 7 is not required, the information processing apparatus 3-2 may be capable of shortening the time required for estimating the viewpoint difference as compared with the information processing apparatus 3.

### Modification Example 3 of Information Processing Apparatus 3

The example has been described above in which the virtual endoscopic image 6 is used as the input data of the viewpoint difference learning data instead of the endoscopic image 4 from the viewpoint of easily preparing the viewpoint difference learning data. However, in a case where the viewpoint of the endoscope 2 can be acquired by devising sensors that measure the viewpoint of the endoscope 2, a viewpoint difference estimation model 22C that outputs the viewpoint difference between the endoscopic image 4 and the virtual endoscopic image 6 using the endoscopic image 4 is generated by machine learning.

Fig. 17 is a diagram showing an example of a method for estimating the viewpoint of the endoscope 2 in an information processing apparatus 3-3 which is a modification example of the information processing apparatus 3. An example of a functional configuration of the information processing apparatus 3-3 is the same as the example of the functional configuration of the information processing apparatus 3 shown in Fig. 1, and the information processing apparatus 3-3 is configured by the computer shown in Fig. 12.

The information processing apparatus 3-3 estimates the viewpoint difference using the viewpoint difference estimation model 22C that, in a case where the virtual endoscopic image 6, the mask image 8, and the endoscopic image 4 (hereinafter, referred to as a "mask superimposed endoscopic image 4A") on which the mask image 8 has been superimposed are input, outputs the viewpoint difference between the endoscopic image 4, which is a generation source of the mask superimposed endoscopic image 4A, and the virtual endoscopic image 6.

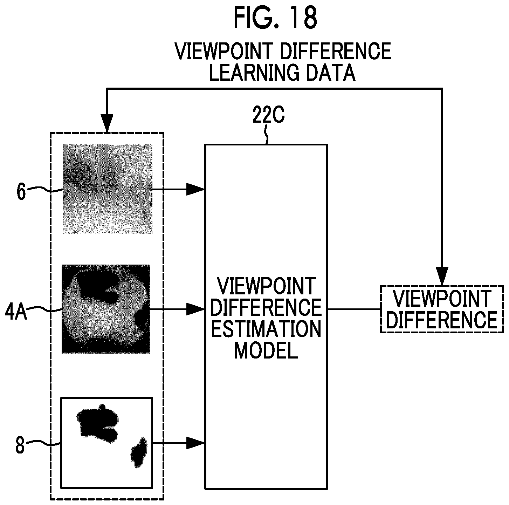

Fig. 18 is a diagram showing an example of the machine learning of the viewpoint difference estimation model 22C. As shown in Fig. 18, a plurality of viewpoint difference learning data items, in which the viewpoint difference between the mask superimposed endoscopic image 4A having the positional information of the unknown structure region superimposed thereon and the virtual endoscopic image 6 having a viewpoint different from that of the mask superimposed endoscopic image 4A is associated with the mask superimposed endoscopic image 4A, the virtual endoscopic image 6, and the mask image 8 indicating the positional information of the unknown structure region, are prepared in advance, and the machine learning of the viewpoint difference estimation model 22C is performed such that, in a case where the mask superimposed endoscopic image 4A, the virtual endoscopic image 6, and the mask image 8 included in the viewpoint difference learning data are input, the viewpoint difference estimation model 22C outputs the viewpoint difference associated with each input. That is, in the viewpoint difference learning data of the viewpoint difference estimation model 22C, the mask superimposed endoscopic image 4A, the virtual endoscopic image 6, and the mask image 8 are input data, and the viewpoint difference is training data.

It is preferable to use the positional information of the actual unknown structure region included in the endoscopic image 4, which is the generation source of the mask superimposed endoscopic image 4A, for the mask image 8 in the viewpoint difference learning data. However, the positional information of the randomly set unknown structure region may be used. **In** addition, an image obtained by adding the positional information of the randomly set unknown structure region to the positional information of the actual unknown structure region included in the endoscopic image 4 may be used as the mask image 8 in the viewpoint difference learning data.

According to the information processing apparatus 3-3 that estimates the viewpoint difference using the viewpoint difference estimation model 22C, the processing of inputting the endoscopic image 4 to the image conversion model 20 and converting the endoscopic image 4 into the pseudo virtual endoscopic image 7 is not necessary. Therefore, the information processing apparatus 3-3 may be capable of shortening the time required for estimating the viewpoint difference as compared to the information processing apparatus 3.

**In** the case of the example of the machine learning of the viewpoint difference estimation model 22C shown in Fig. 18, the mask superimposed endoscopic image 4A generated from the actual endoscopic image 4 is used as the input data of the viewpoint difference learning data. However, in the case of the endoscope 2 in which it is difficult to acquire the viewpoint, it is difficult to perform the machine learning of the viewpoint difference estimation model 22C using the method shown in Fig. 18. **In** addition, the mask superimposed endoscopic image 4A generated from a larger number of endoscopic images 4 captured from various viewpoints is required in order to improve the accuracy of estimation of the viewpoint difference by the viewpoint difference estimation model 22C. However, it is difficult to change the viewpoint of the endoscope 2 in the bronchus in various directions, and the endoscopic image 4 can be captured only in the actual endoscopy. Therefore, the opportunity for imaging is also limited.

Therefore, the machine learning of the viewpoint difference estimation model 22C may be performed by generating the pseudo endoscopic image 17 corresponding to the endoscopic image 4 from the virtual endoscopic image 6 and by using the pseudo endoscopic image 17 instead of the endoscopic image 4. The pseudo endoscopic image 17 is an image obtained by converting the virtual endoscopic image 6 into an expression format that looks like the endoscopic image 4. Specifically, the pseudo endoscopic image 17 is an image obtained by adding the texture of the bronchus or the unknown structure region to the virtual endoscopic image 6 showing the shape of the bronchus such that the image looks like the actual endoscopic image 4.

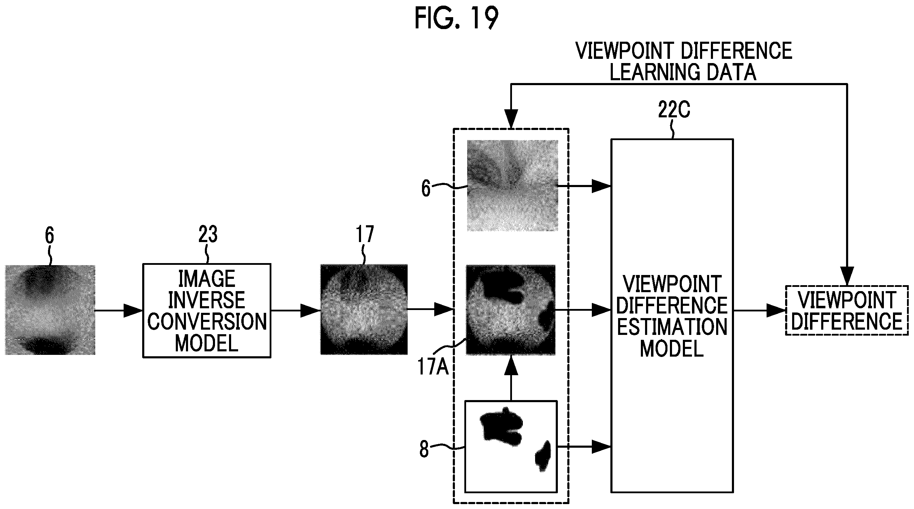

Fig. 19 is a diagram showing an example of the machine learning of the viewpoint difference estimation model 22C using the pseudo endoscopic image 17. As shown in Fig. 19, two virtual endoscopic images 6 having different viewpoints are prepared, and one virtual endoscopic image 6 is converted into the pseudo endoscopic image 17 using an image inverse conversion model 23. Furthermore, the mask image 8 is superimposed on the generated pseudo endoscopic image 17, and the positional information of the unknown structure region is superimposed on the pseudo endoscopic image 17 to generate a mask superimposed pseudo endoscopic image 17A. In addition, since the mask superimposed pseudo endoscopic image 17A is an image generated based on one of the two virtual endoscopic images 6 generated from the bronchial model 5, the viewpoint difference from the other virtual endoscopic image 6 is known.

A plurality of viewpoint difference learning data items, in which the viewpoint difference between the virtual endoscopic image 6 and the mask superimposed pseudo endoscopic image 17A is associated with the virtual endoscopic image 6, the mask superimposed pseudo endoscopic image 17A, and the mask image 8 indicating the positional information of the unknown structure region, are prepared in advance, and the machine learning of the viewpoint difference estimation model 22C is performed such that, in a case where the virtual endoscopic image 6, the mask superimposed pseudo endoscopic image 17A, and the mask image 8 included in the viewpoint difference learning data are input, the viewpoint difference estimation model 22C outputs the viewpoint difference associated with each input. That is, in the viewpoint difference learning data of the viewpoint difference estimation model 22C, the virtual endoscopic image 6, the mask superimposed pseudo endoscopic image 17A, and the mask image 8 are input data, and the viewpoint difference is training data.

The positional information of the randomly set unknown structure region is set in the mask image 8 in the viewpoint difference learning data of the viewpoint difference estimation model 22C shown in Fig. 19.

In the example of the machine learning of the viewpoint difference estimation model 22C shown in Fig. 19, it is possible to prepare a larger amount of viewpoint difference learning data having various viewpoint differences regardless of the type of the endoscope 2, as compared to the example of the machine learning of the viewpoint difference estimation model 22C shown in Fig. 18.

### Second Embodiment

In the first embodiment, the example has been described in which the viewpoint difference is estimated using the viewpoint difference estimation model 22 generated by machine learning and the viewpoint difference estimation models 22A to 22C generated using the viewpoint difference learning data including the input data of a different type from the viewpoint difference learning data used for the machine learning of the viewpoint difference estimation model 22 and the viewpoint of the endoscope 2 is estimated based on the estimated viewpoint difference.

However, a method for estimating the viewpoint of the endoscope 2 is not limited to the method using the viewpoint difference estimation model 22 and the viewpoint difference estimation models 22A to 22C generated by machine learning.

In the second embodiment, an information processing apparatus 3-4 that estimates a viewpoint difference between images based on a similarity between the images and estimates the viewpoint of the endoscope 2 based on the estimated viewpoint difference will be described.

An example of a functional configuration of the information processing apparatus 3-4 according to the second embodiment is the same as the example of the functional configuration of the information processing apparatus 3 shown in Fig. 1, and the information processing apparatus 3-4 is configured by the computer shown in Fig. 12.

The information processing apparatus 3-4 estimates the viewpoint of the endoscope 2 from a similarity between a depth image (hereinafter, referred to as an "endoscopic depth image 18") corresponding to the endoscopic image 4 and a depth image generated using the bronchial model 5. The similarity is represented by, for example, a numerical value. For example, it is assumed that, as the numerical value of the similarity is larger, the similarity between the images is higher.

Therefore, the information processing apparatus 3-4 generates the endoscopic depth image 18. Specifically, the information processing apparatus 3-4 generates the endoscopic depth image 18 in which the endoscopic image 4 captured by the endoscope 2 is represented as distance data from an entrance of the bronchus, using, for example, the distance data obtained from a depth sensor that measures the distance from the entrance of the bronchus. The endoscopic depth image 18 is an example of the depth image corresponding to the endoscopic image 4. In addition, for example, a stereo camera, a time-of-flight (TOF) sensor, a light detection and ranging (LiDAR) sensor, or a photoelectric sensor is used as the depth sensor.

Further, the information processing apparatus 3-4 superimposes the positional information of the unknown structure region detected from the endoscopic image 4 on the generated endoscopic depth image 18 to generate a mask superimposed endoscopic depth image 18A. The mask superimposed endoscopic depth image 18A is an example of a superimposed depth image.

In addition, the information processing apparatus 3-4 generates a depth image (hereinafter, referred to as a "virtual depth image 19") at respective positions inside the bronchial model 5.

Furthermore, the information processing apparatus 3-4 generates a mask superimposed virtual depth image 19A obtained by superimposing the positional information of the unknown structure region detected from the endoscopic image 4 on each generated virtual depth image 19. The mask superimposed virtual depth image 19A is an example of the superimposed virtual depth image.

The information processing apparatus 3-4 calculates a similarity between each of the generated mask superimposed virtual depth images 19A and the mask superimposed endoscopic depth image 18A and specifies a mask superimposed virtual depth image 19A that is most similar to the mask superimposed endoscopic depth image 18A. In addition, the information processing apparatus 3-4 searches for the viewpoint at which the similarity with the endoscopic image 4, which is the generation source of the mask superimposed endoscopic depth image 18A, has the maximum value at the position indicated by the virtual depth image 19, which is the generation source of the specified mask superimposed virtual depth image 19A, and estimates the viewpoint of the endoscope 2. That is, the viewpoint at which the similarity with the endoscopic image 4 corresponding to the mask superimposed endoscopic depth image 18A has the maximum value at the position indicated by the mask superimposed virtual depth image 19A most similar to the mask superimposed endoscopic depth image 18A is the viewpoint of the endoscope 2.

In addition, for example, a known image recognition method that compares feature amounts of images and calculates the similarity can be applied to calculate the similarity between the mask superimposed endoscopic depth image 18A and the mask superimposed virtual depth image 19A. In this case, it is preferable that the unknown structure regions in the mask superimposed endoscopic depth image 18A and the mask superimposed virtual depth image 19A are not used for the calculation of the similarity or are treated as images with the same pixel value to reduce the influence of the unknown structure region on the calculation of the similarity between the images.

Further, a similarity estimation model that is generated by machine learning and outputs the similarity between two images may be used to calculate the similarity between the images.

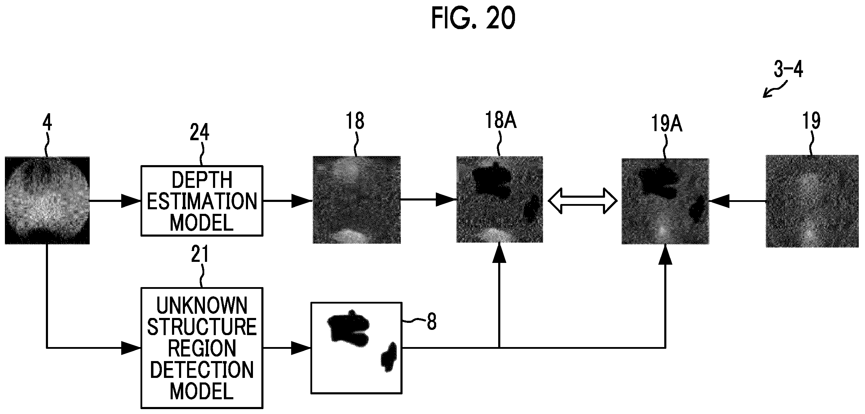

Fig. 20 is a diagram showing an example of a method for estimating the viewpoint of the endoscope 2 in the estimation unit 3F of the information processing apparatus 3-4. For example, the estimation unit 3F inputs the endoscopic image 4 to a depth estimation model 24 to convert the endoscopic image 4 into the endoscopic depth image 18.

The depth estimation model 24 is, for example, a model generated in advance using a GAN that has been trained to output the endoscopic depth image 18 from the endoscopic image 4 and is stored in advance in the storage unit 3D.

Further, the estimation unit 3F superimposes the mask image 8 generated by the unknown structure region detection model 21 on the endoscopic depth image 18 generated by the depth estimation model 24 to generate the mask superimposed endoscopic depth image 18A and superimposes the mask image 8 on the virtual depth image 19 at each position generated by the generation unit 3E using the bronchial model 5 to generates the mask superimposed virtual depth image 19A.

The estimation unit 3F calculates the similarity between each generated mask superimposed virtual depth image 19A and the mask superimposed endoscopic depth image 18A to estimate the viewpoint of the endoscope 2.

Next, a process of the information processing apparatus 3-4 that estimates the viewpoint of the endoscope 2 will be described in detail.

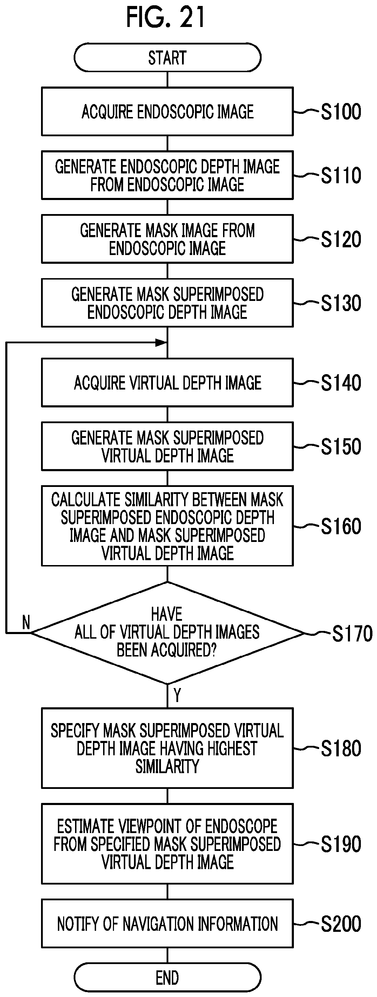

Fig. 21 is a flowchart showing an example of a flow of an estimation process executed by the information processing apparatus 3-4 in a case where an instruction to start the estimation of the viewpoint of the endoscope 2 is received by the operation of the operation unit 13 by the medical worker. The CPU 10A of the information processing apparatus 3-4 reads the control program 11 from the ROM 10B and executes the estimation process. It is assumed that the storage unit 15 stores the bronchial model 5 of the subject, the virtual depth image 19 at respective positions in the bronchial model 5 of the subject, the unknown structure region detection model 21, and the depth estimation model 24 in advance.

First, in Step S100, the CPU 10A acquires the endoscopic image 4 from the endoscope 2 via the I/F unit 12.

In Step S110, the CPU 10A inputs the endoscopic image 4 acquired by the process in Step S100 to the depth estimation model 24 to generate the endoscopic depth image 18.

In Step S120, the CPU 10A inputs the endoscopic image 4 acquired by the process in Step S100 to the unknown structure region detection model 21 to generate the mask image 8.

In Step S130, the CPU 10A superimposes the mask image 8 generated by the process in Step S120 on the endoscopic depth image 18 generated by the process in Step S110 to generate the mask superimposed endoscopic depth image 18A.

In Step S140, the CPU 10A acquires any one virtual depth image 19 from the plurality of virtual depth images 19 stored in the storage unit 15.

In Step S150, the CPU 10A superimposes the mask image 8 generated by the process in Step S120 on the virtual depth image 19 acquired by the process in Step S140 to generate the mask superimposed virtual depth image 19A.

In Step S160, the CPU 10A calculates the similarity between the mask superimposed endoscopic depth image 18A generated by the process in Step S130 and the mask superimposed virtual depth image 19A generated by the process in Step S150 and stores the calculated similarity in the RAM 10C in association with the mask superimposed virtual depth image 19A used to calculate the similarity.

In Step S170, the CPU 10A determines whether or not all of the virtual depth images 19 stored in the storage unit 15 have been acquired. In a case where all of the virtual depth images 19 have not been acquired, the CPU 10A proceeds to Step S140 and acquires one virtual depth image 19 that has not been acquired. That is, the CPU 10A repeats the process in Steps S140 to S170 until it is determined in the determination process in Step S170 that all of the virtual depth images 19 have been acquired and associates the similarity with the mask superimposed endoscopic depth image 18A with each mask superimposed virtual depth image 19A.

On the other hand, in a case where it is determined in the determination process in Step S170 that all of the virtual depth images 19 have been acquired, the CPU 10A proceeds to Step S180.

In Step S180, the CPU 10A specifies the mask superimposed virtual depth image 19A associated with the highest similarity among all of the mask superimposed virtual depth images 19A as the mask superimposed virtual depth image 19A most similar to the mask superimposed endoscopic depth image 18A with reference to the similarities associated with each mask superimposed virtual depth image 19A stored in the RAM 10C.

In Step S190, the CPU 10A searches for a viewpoint at which the similarity with the mask superimposed endoscopic depth image 18A has the maximum value at the position of the bronchial model 5 represented by the virtual depth image 19, which is the generation source of the mask superimposed virtual depth image 19A specified by the process in Step S180, and estimates the viewpoint of the endoscope 2.

In Step S200, the CPU 10A generates the virtual endoscopic image 6 in a case where the bronchial model 5 is viewed from the viewpoint estimated by the process in Step S190 and displays the generated virtual endoscopic image 6 side by side with, for example, the endoscopic image 4 acquired by the process in Step S100 on the display. Therefore, the virtual endoscopic image 6 corresponding to the viewpoint of the endoscope 2 is displayed according to a change in the viewpoint of the endoscope 2.

In addition, the CPU 10A searches for a route from the estimated viewpoint of the endoscope 2 to the target location using the bronchial model 5.

The CPU 10A notifies the medical worker of the traveling direction of the endoscope 2, such as a "right direction" or a "downward direction", by voice through the speaker based on the search result of the route. In this way, the estimation process shown in Fig. 21 is ended.

The example in which the viewpoint of the endoscope 2 is estimated from the similarity between the mask superimposed endoscopic depth image 18A and the mask superimposed virtual depth image 19A has been described above. However, the objects between which the similarity is to be calculated are not limited to the depth images.

The information processing apparatus 3-4 may estimate the viewpoint of the endoscope 2, for example, from the similarity between the mask superimposed endoscopic image 4A and the virtual endoscopic image 6A on which the positional information of the unknown structure region indicated by the mask image 8 has been superimposed.

Specifically, the generation unit 3E generates the virtual endoscopic image 6 at respective positions in the bronchial model 5 in advance. The estimation unit 3F superimposes the mask image 8 on each virtual endoscopic image 6 to generate the virtual endoscopic image 6A, calculates the similarity between the virtual endoscopic image 6A, on which the positional information of the unknown structure region has been superimposed, and the mask superimposed endoscopic image 4A, and specifies the virtual endoscopic image 6A most similar to the mask superimposed endoscopic image 4A among the plurality of virtual endoscopic images 6A. In addition, the estimation unit 3F may search for a viewpoint at which the similarity with the mask superimposed endoscopic image 4A or the endoscopic image 4, which is the generation source of the mask superimposed endoscopic image 4Ahas the maximum value at the position indicated by the virtual endoscopic image 6, which is the generation source of the specified virtual endoscopic image 6A, and may estimate the viewpoint of the endoscope 2.

As described above, according to the information processing apparatus 3-4 of the second embodiment, the viewpoint of the endoscope 2 is estimated by calculating the similarity between the image, which includes the positional information of the unknown structure region included in the endoscopic image 4 and has been generated from the endoscopic image 4, and the image which includes the positional information of the unknown structure region included in the endoscopic image 4 and has been generated from the bronchial model 5. Therefore, in the first embodiment, the viewpoint difference learning data for generating the viewpoint difference estimation models 22, 22A, and 22C used for estimating the viewpoint difference may not be generated in advance.

In addition, supplementary information related to the position of the endoscope 2 may be added as the input data of the viewpoint difference learning data of the viewpoint difference estimation models 22 and 22A to 22C (collectively referred to as "each viewpoint difference estimation model 22" for convenience of description) in the information processing apparatus 3 and the information processing apparatuses 3-1 to 3-3 (collectively referred to as "each information processing apparatus 3" for convenience of description) according to the first embodiment. **In** this case, each information processing apparatus 3 estimates the viewpoint of the endoscope 2 using the endoscopic image 4, the positional information of the unknown structure region included in the endoscopic image 4, the virtual endoscopic image 6, the supplementary information, and each viewpoint difference estimation model 22.

For example, a depth image corresponding to the endoscopic image 4 is used as the supplementary information. In addition, an insertion length of the endoscope 2 into the bronchus at the time when the endoscopic image 4 is captured may be used as the supplementary information.

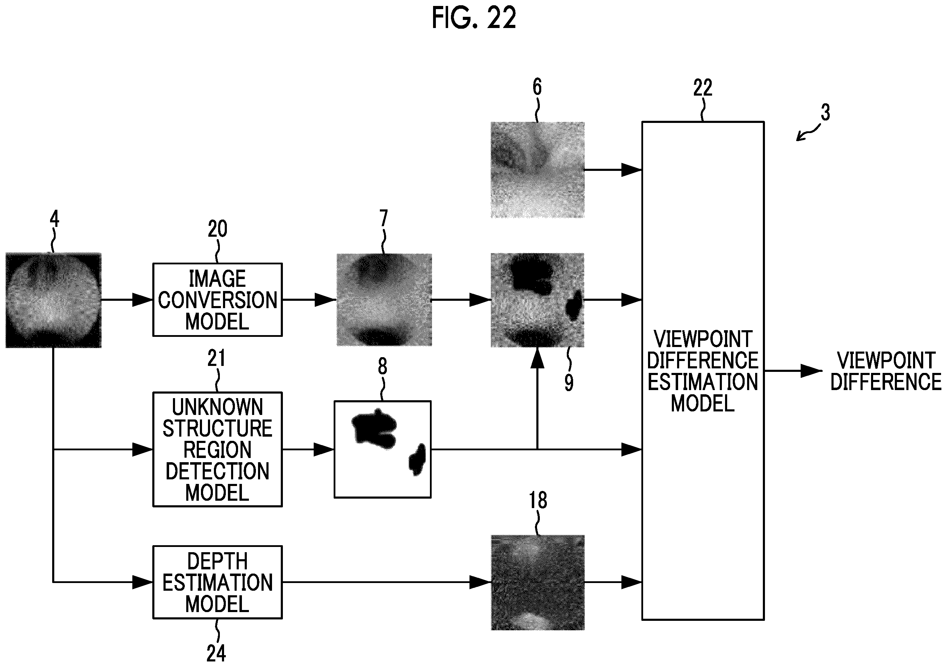

Fig. 22 is a diagram showing an example of a situation in which the supplementary information is added in the method for estimating the viewpoint difference of the endoscope 2 shown in Fig. 6. In the example shown in Fig. 22, the endoscopic depth image 18 generated from the endoscopic image 4 by the depth estimation model 24 is input as the supplementary information to the viewpoint difference estimation model 22.

**In** a case where the supplementary information is added to the input of each viewpoint difference estimation model 22, the accuracy of estimation of the viewpoint may be improved as compared to a case where the viewpoint of the endoscope 2 is estimated without using the supplementary information.

One form of the information processing system 1 has been described above using the embodiment. However, the disclosed form of the information processing system 1 is an example. The form of the information processing system 1 is not limited to the range described in the embodiment. Various modifications and improvements can be added to the embodiments without departing from the gist of the present disclosure, and the embodiments to which the modifications or improvements are added are also included in the technical scope of the present disclosure.

For example, the internal processing order in the flowchart of each estimation process shown in Figs. 13 and 21 may be changed without departing from the gist of the present disclosure.

In each of the above-described embodiments, the form in which each estimation process is implemented by software processing has been described as an example. However, the same processes as those in the flowcharts of each estimation process may be performed by hardware. In this case, the processing speed can be increased as compared to a case where each estimation process is implemented by the software processing.

In the above-described embodiments, the term "processor" refers to hardware in a broad sense. Examples of the processor include general processors (for example, the CPU 10A) and dedicated processors (for example, a graphics processing unit: GPU, an application specific integrated circuit: ASIC, a field programmable gate array: FPGA, and a programmable logic device).

Further, the operation of the processor in each of the above-described embodiments may be performed not only by one processor but also by cooperation of a plurality of processors provided at physically separated positions. In addition, the order of the operations of the processor is not limited to only the order described in the embodiments and may be changed as appropriate.

In each of the above-described embodiments, the example in which the control program 11 is stored in the ROM 10B has been described. However, the storage destination of the control program 11 is not limited to the ROM 10B. The control program 11 can also be provided in a form in which the control program 11 is recorded in a computer-readable storage medium.

For example, the control program 11 may be provided in a form in which the control program 11 is recorded in an optical disk such as a compact disk read only memory (CD-ROM), a digital versatile disk read only memory (DVD-ROM), or a Blu-ray disc. In addition, the control program 11 may be provided in a form in which the control program 11 is recorded in a portable semiconductor memory such as a universal serial bus (USB) memory or a memory card. The ROM 10B, the CD-ROM, the DVD-ROM, the Blu-ray disc, the USB, and the memory card are examples of a non-transitory storage medium.

Further, the control unit 10 may download the control program 11 from an external apparatus connected to the communication line via the I/F unit 12 and store the downloaded control program 11 in the ROM 10B of the control unit 10.

For the above-described embodiments, the following supplementary notes are further disclosed.

### Supplementary Note 1

An information processing apparatus comprising:
a detection unit that detects an unknown structure region from an endoscopic image of a living body;
an acquisition unit that acquires a three-dimensional model of the living body generated in advance;
an estimation unit that estimates, as a viewpoint of an endoscope, a posture of the endoscope at a position of the three-dimensional model corresponding to a position where the endoscopic image has been captured, using the endoscopic image, the unknown structure region, and the three-dimensional model; and
a notification unit that notifies of the estimated viewpoint of the endoscope.

### Supplementary Note 2

The information processing apparatus according to Supplementary Note 1, further comprising:
a generation unit that generates a virtual endoscopic image of the living body in a case where the three-dimensional model is viewed from a predetermined position in the three-dimensional model,
wherein the estimation unit inputs the endoscopic image, positional information of the unknown structure region, and the virtual endoscopic image generated from the three-dimensional model to an estimation model that has been subjected to machine learning in advance to output a viewpoint difference between the endoscopic image and the virtual endoscopic image for the endoscopic image, the positional information of the unknown structure region, and the virtual endoscopic image to acquire the viewpoint difference, and estimates the viewpoint of the endoscope from the viewpoint difference and a position at which the virtual endoscopic image has been obtained.

### Supplementary Note 3

The information processing apparatus according to Supplementary Note 2,
wherein the estimation unit estimates the viewpoint of the endoscope, using a pseudo virtual endoscopic image obtained by converting the endoscopic image into an expression format of the virtual endoscopic image generated from the three-dimensional model instead of the endoscopic image.

### Supplementary Note 4

The information processing apparatus according to Supplementary Note 3,
wherein the estimation unit estimates the viewpoint of the endoscope, using an image, on which the positional information of the unknown structure region has been superimposed, as the pseudo virtual endoscopic image.

### Supplementary Note 5

The information processing apparatus according to Supplementary Note 3,
wherein the estimation unit estimates the viewpoint of the endoscope, using the pseudo virtual endoscopic image, on which the positional information of the unknown structure region has been superimposed, as the endoscopic image and the positional information of the unknown structure region.

### Supplementary Note 6

The information processing apparatus according to Supplementary Note 2,
wherein the estimation unit estimates the viewpoint of the endoscope, using the endoscopic image on which the positional information of the unknown structure region has been superimposed.

### Supplementary Note 7

The information processing apparatus according to any one of Supplementary Notes 2 to 6,
wherein the endoscopic image used for the machine learning of the estimation model is an image generated from the virtual endoscopic image.

### Supplementary Note 8

The information processing apparatus according to Supplementary Note 2,
wherein the estimation model is a model that has been subjected to machine learning in advance to output the viewpoint difference for the endoscopic image, the positional information of the unknown structure region, the virtual endoscopic image, and supplementary information related to a position of the endoscope, and
the estimation unit inputs the endoscopic image, the positional information of the unknown structure region, the virtual endoscopic image, and the supplementary information to the estimation model to estimate the viewpoint of the endoscope.

### Supplementary Note 9

The information processing apparatus according to Supplementary Note 8,
wherein the supplementary information is a depth image corresponding to the endoscopic image.

### Supplementary Note 10

The information processing apparatus according to Supplementary Note 1,
wherein the estimation unit superimposes positional information of the unknown structure region on a depth image corresponding to the endoscopic image to generate a superimposed depth image, superimposes the positional information of the unknown structure region on virtual depth images at respective positions in the three-dimensional model to generate superimposed virtual depth images, and estimates the viewpoint of the endoscope from a position indicated by the superimposed virtual depth image most similar to the superimposed depth image among the superimposed virtual depth images at respective positions in the three-dimensional model.

### Supplementary Note 11

The information processing apparatus according to Supplementary Note 1,
wherein the estimation unit generates the endoscopic image, on which positional information of the unknown structure region in the endoscopic image has been superimposed, and virtual endoscopic images of the living body in a case where the three-dimensional model is viewed from respective positions in the three-dimensional model, each of which has the positional information of the unknown structure region in the endoscopic image superimposed thereon, and estimates the viewpoint of the endoscope from a position indicated by the virtual endoscopic image most similar to the endoscopic image, on which the positional information of the unknown structure region has been superimposed, among the virtual endoscopic images at respective positions in the three-dimensional model.

### Supplementary Note 12

The information processing apparatus according to any one of Supplementary Notes 1 to 11,
wherein the notification unit notifies of the unknown structure region detected from the endoscopic image.

### Supplementary Note 13

The information processing apparatus according to Supplementary Note 12,
wherein the notification unit surrounds the unknown structure region in the endoscopic image with a frame to notify of the unknown structure region.

### Supplementary Note 14

The information processing apparatus described in Supplementary Note 12,
wherein the notification unit displays an edge of the endoscopic image, whose distance from the unknown structure region is equal to or less than a predetermined value, in a form different from other edges of the endoscopic image.

### Supplementary Note 15

The information processing apparatus according to Supplementary Note 13 or 14,
wherein the notification unit notifies of the unknown structure region in a case where a proportion of the unknown structure region to the endoscopic image is equal to or greater than a prescribed proportion.

### Supplementary Note 16

An information processing program causing a computer to execute a process comprising:
detecting an unknown structure region from an endoscopic image of a living body;
acquiring a three-dimensional model of the living body generated in advance;
estimating, as a viewpoint of an endoscope, a posture of the endoscope at a position of the three-dimensional model corresponding to a position where the endoscopic image has been captured, using the endoscopic image, the unknown structure region, and the three-dimensional model; and
notifying of the estimated viewpoint of the endoscope.

## Claims

1. An information processing apparatus (3) comprising:
a detection unit (3B) that detects an unknown structure region from an endoscopic image (4) of a living body;
an acquisition unit (3C) that acquires a three-dimensional model (5) of the living body generated in advance;
an estimation unit (3F) that estimates, as a viewpoint of an endoscope (2), a position and a posture of the endoscope on the three-dimensional model (5) corresponding to a position where the endoscopic image (4) has been captured, using the endoscopic image (4), the unknown structure region, and the three-dimensional model (5); and
a notification unit (3G) that notifies of the estimated viewpoint of the endoscope (2).

2. The information processing apparatus (3) according to claim 1, further comprising:
a generation unit (3E) that generates a virtual endoscopic image (6, 6A) of the living body in a case where the three-dimensional model (5) is viewed from a predetermined position in the three-dimensional model (5),
wherein the estimation unit (3F) inputs the endoscopic image (4), positional information of the unknown structure region, and the virtual endoscopic image (6, 6A) generated from the three-dimensional model (5) to an estimation model (22) that has been subjected to machine learning in advance to output a viewpoint difference between the endoscopic image (4) and the virtual endoscopic image (6, 6A) for the endoscopic image (4), the positional information of the unknown structure region, and the virtual endoscopic image (6, 6A) to acquire the viewpoint difference, and estimates the viewpoint of the endoscope (2) from the viewpoint difference and a position at which the virtual endoscopic image (6, 6A) has been obtained.

3. The information processing apparatus (3) according to claim 2,
wherein the estimation unit (3F) estimates the viewpoint of the endoscope (2), using a pseudo virtual endoscopic image obtained by converting the endoscopic image (4) into an expression format of the virtual endoscopic image (6, 6A) generated from the three-dimensional model (5) instead of the endoscopic image (4).

4. The information processing apparatus (3) according to claim 3,
wherein the estimation unit (3F) estimates the viewpoint of the endoscope (2), using an image, on which the positional information of the unknown structure region has been superimposed, as the pseudo virtual endoscopic image (6, 6A).

5. The information processing apparatus (3) according to claim 3,
wherein the estimation unit (3F) estimates the viewpoint of the endoscope (2), using the pseudo virtual endoscopic image (7), on which the positional information of the unknown structure region has been superimposed, as the endoscopic image (4) and the positional information of the unknown structure region.

6. The information processing apparatus (3) according to claim 2,
wherein the estimation unit estimates the viewpoint of the endoscope, using the endoscopic image on which the positional information of the unknown structure region has been superimposed.

7. The information processing apparatus (3) according to claim 2,
wherein the endoscopic image (4) used for the machine learning of the estimation model (22) is an image generated from the virtual endoscopic image (6, 6A).

8. The information processing apparatus (3) according to claim 2,
wherein the estimation model (22) is a model that has been subjected to machine learning in advance to output the viewpoint difference for the endoscopic image (4), the positional information of the unknown structure region, the virtual endoscopic image (6, 6A), and supplementary information related to a position of the endoscope (2), and
the estimation unit (3F) inputs the endoscopic image (4), the positional information of the unknown structure region, the virtual endoscopic image (6, 6A), and the supplementary information to the estimation model (22) to estimate the viewpoint of the endoscope (2).

9. The information processing apparatus (3) according to claim 8,
wherein the supplementary information is a depth image corresponding to the endoscopic image (4).

10. The information processing apparatus (3) according to claim 1,
wherein the estimation unit (3F) superimposes positional information of the unknown structure region on a depth image corresponding to the endoscopic image (4) to generate a superimposed depth image (18A), superimposes the positional information of the unknown structure region on virtual depth images (19) at respective positions in the three-dimensional model (5) to generate superimposed virtual depth images (19A), and estimates the viewpoint of the endoscope (2) from a position indicated by the superimposed virtual depth image (19A) most similar to the superimposed depth image (18A) among the superimposed virtual depth images (19A) at respective positions in the three-dimensional model (5).

11. The information processing apparatus (3) according to claim 1,
wherein the estimation unit (3F) generates the endoscopic image (4), on which positional information of the unknown structure region in the endoscopic image (4) has been superimposed, and virtual endoscopic images (6, 6A) of the living body in a case where the three-dimensional model (5) is viewed from respective positions in the three-dimensional model (5), each of which has the positional information of the unknown structure region in the endoscopic image superimposed thereon, and estimates the viewpoint of the endoscope (2) from a position indicated by the virtual endoscopic image (6, 6A) most similar to the endoscopic image (4), on which the positional information of the unknown structure region has been superimposed, among the virtual endoscopic images (6, 6A) at respective positions in the three-dimensional model (5).

12. The information processing apparatus (3) according to any one of claims 1 to 11,
wherein the notification unit (3G) notifies of the unknown structure region detected from the endoscopic image (4).

13. The information processing apparatus (3) according to claim 12,
wherein the notification unit (3G) surrounds the unknown structure region in the endoscopic image (4) with a frame to notify of the unknown structure region.

14. The information processing apparatus (3) according to claim 12,
wherein the notification unit (3G) displays an edge of the endoscopic image (4), whose distance from the unknown structure region is equal to or less than a predetermined value, in a form different from other edges of the endoscopic image (4).

15. The information processing apparatus (3) according to claim 13 or 14,
wherein the notification unit (3G) notifies of the unknown structure region in a case where a proportion of the unknown structure region to the endoscopic image (4) is equal to or greater than a prescribed proportion.

16. A storage medium (10B) storing an information processing program (11) executable by a computer (10) to execute a process comprising:
detecting an unknown structure region from an endoscopic image (4) of a living body;
acquiring a three-dimensional model (5) of the living body generated in advance;
estimating, as a viewpoint of an endoscope (2), a position and a posture of the endoscope (2) on the three-dimensional model (5) corresponding to a position where the endoscopic image (4) has been captured, using the endoscopic image (4), the unknown structure region, and the three-dimensional model (5); and
notifying of the estimated viewpoint of the endoscope (2).
